# EUROPEAN PATENT SPECIFICATION

(11) **EP 0 301 856 B1**
(45) Date of publication and mention of the grant of the patent: **24.05.1995**
(21) Application number: 88306958.5
(22) Date of filing: 28.07.1988
(51) Int. Cl.: A61K 9/30, A61K 9/22

(54) **Delivery system**
Verabreichungsformen für Pharmaka
Dispositif libérant des produits pharmaceutiques

(30) Priority: 28.07.1987 US 78534
(43) Date of publication of application: 01.02.1989
(73) Proprietor: BIOMEASURE, INC., Hopkinton Massachusetts 01748 (US)
(72) Inventor: Moreau, Jacques-Pierre, Upton Massachusetts 01568 (US); Kitchell, Judith P., Newton Massachusetts 02166 (US)
(74) Representative: Deans, Michael John Percy

(56) References cited:
- EP-A- 0 206 890
- EP-A- 0 263 083
- WO-A-87/04070
- DE-A- 2 824 112
- US-A- 3 854 480
- US-A- 4 351 337
- US-A- 4 622 244

## Description

This invention is concerned with release of agents, such as therapeutic agents, or other substances from delivery systems adapted for delivery by parenteral injection or implantation, and provides novel delivery systems, methods of administrating such agents or substances to vertebrates other than during surgical treatment or in therapy or in a method of diagnosis, and methods of providing such delivery systems.

Biodegradable polymers containing a therapeutic agent are often used to administer the agent to a patient. Generally, the release results from the dissolution of the polymer and from the diffusion of the agent through pores and channels in the matrix.

Delivery systems consisting of a biodegradable polymer and a therapeutic agent and adapted for parenteral injection or implantation are well known and can be made in a variety of ways and in a number of different forms (see for example US-A-4351337 Sidman).

Such delivery systems ("implants" as they are referred to by Sidman) suffer from a significant problem, namely that of an often unwanted initial burst of release of the agent immediately following parenteral injection or implantation. Sidman seeks to overcome this problem by overcoating his initially formed implants comprising the biodegradable polymer and the therapeutic agent with a layer of pure polymer but finds that a single layer of such polymer is inadequate to solve the problem. Sidman resorts to the complicated procedure of forming repetitive overcoated layers of pure polymer in order to avoid this initial burst of release of the agent.

The present invention is concerned with solving the problem of the unwanted initial burst of released agent by a simple procedure which avoids the need for repeated and complicated process steps such as those necessary to form multiple overcoat layers of pure polymer. It is the applicants' belief that prior to the present invention this longstanding problem has not been susceptible to solution.

Typical of processes for the manufacture of delivery systems comprising a biodegradable polymer and a therapeutic agent and adapted for parenteral injection or implantation are solvent casting, coacervation procedures and dispersion. When the former procedure is used, the agent is dispersed as small particles throughout the system. The small particles are irregularly shaped with small jagged edges; the edges may extend to the surface of the system, or may cause cracks in the system that extend to the surface. It is believed that when such a system is inserted into a patient, a dose of agent larger than the desired dosage is released from the system through these surface contacts.

Delivery systems prepared by coacervation consist of an aggregate of agent surrounded by the polymer. The large crystal may have jagged edges extending to the surface, or may cause cracks that extend to the surface. Accordingly, the initial burst of agent problem is also present in these systems.

According to a first aspect of the present invention, there is provided a delivery system adapted for delivery of an agent, such as a therapeutic agent, or other substance to a living person or animal by parenteral injection or implantation, comprising a biodegradable polymer and a therapeutic agent dispersed in said polymer, and being coated with a barrier substance that is effective, during a period of forty-eight hours immediately subsequent to parenteral injection or implantation of said system into a living person or animal, to decrease the quantity of said agent or substance released from said system, as compared with the quantity of said agent or substance released from a similar said system not so coated, during said period, said barrier substance being not susceptible to enzymatic attack and one which dissipates in said living person or animal substantially by wearing off from the surface of said delivery system.

In a second and alternative aspect thereof, the invention provides a method of providing a delivery system adapted for delivery of an agent, such as a therapeutic agent, or other substance to a living person or animal by parenteral injection or implantation, characterised in comprising the steps of: coating a delivery system comprising a biodegradable polymer and said agent or substance dispersed within said polymer with a barrier substance effective, during a period of forty-eight hours immediately subsequent to the parenteral injection or implantation of said system into a living person or animal, to decrease the quantity of said agent or substance released from said system, as compared with the quantity of said agent or substance released from a similar said system not so coated during said period, said barrier substance being not readily susceptible to enzymatic attack and one which dissipates in said living person or animal substantially by wearing off from the surface of said delivery system; and treating said coated delivery system to remove some but not all of said barrier substance.

The barrier substance may comprise, for example, silicone oils, paraffins or beeswax. Paraffin waxes and beeswax have been suggested as ingredients of enteric coatings to be used to coat drug-containing granules, pellets and tablets intended to be taken by mouth (WO 87/04070 Research Corporation which proposes coatings formed as an emulsion comprising a wax/lipid, an emulsifying agent and water). It is the applicants' belief, however, that their aforementioned barrier substances have never previously been suggested for use in delivery systems adapted for parenteral injection or implantation and comprising a biodegradable polymer and a therapeutic agent.

The applicants have found that coating the surface of such a delivery system with a barrier substance as taught herein limits the release of agent from the delivery system for the initial one or two day period following administration, thus decreasing the agent's possible deleterious side effects that can result from initial bursts
The delivery system is easy to use and inexpensive to make. Moreover, because of the coating, the delivery system has superior handling properties in that it does not clump together when in powdered form.

In the preferred arrangement the barrier substance is a silicone oil preferably with a viscosity of between 10² centipoise and 10⁴ centipoise, and most preferably with a viscosity between 500 centipoise and 2000 centipoise.

The agent or substance concerned need not be for use during surgical treatment or in therapy or in a method of diagnosis of disease in the recipient. The invention accordingly provides, in a third aspect thereof, a method of administering an agent or substance to a vertebrate other than during surgical treatment or in therapy or in a method of diagnosis of disease by the use of a delivery system comprising a biodegradable polymer and said agent or substance, characterised in that it includes the steps of: coating said delivery system with a barrier substance; and injecting or implanting the coated delivery system into said vertebrate; said barrier substance being effective to delay biodegradation of said polymer and to limit the initial release of said agent or substance from said system in comparison with the initial release obtained from a similar said system not coated with said barrier substance.

The invention is hereinafter more particularly described by way of example with reference to release of therapeutic agents.

The delivery system employs a biodegradable polymer containing a therapeutic agent. The system is coated with a barrier substance; the barrier substance, when the system is introduced into a vertebrate (preferably a mammal such as a human or domestic animal such as a dog or cow) decreases the initial burst of therapeutic agent from the system.

A biodegradable polymer is a polymer which slowly dissolves or degrades in a physiological environment into low molecular weight molecules that are then transported from the site. Types of biodegradable polymers suitable for use in a delivery system include polyanhydrides, partially crosslinked proteins, polylactic acid, polyglycolic acid, polyorthoesters, polysaccharides, polaxomers, hydroxypropylcellulose, polyethyleneglycol, copolymers of lactide and glycolide, arid carboxymethylcellulose. Some representative examples of poly(lactide/glycolide) biodegradable polymers are described by Kitchell & Wise, 112 Methods In Enzymology 436 (1985).

The delivery systems produced in accordance with the teaching of the present invention preferably are injected or implanted parenterally into an animal or a human. Where the system is to be injected, it should be small enough in diameter to fit through the needle tip of the syringe. The preferred delivery systems for injection have an average size of 500 microns or less (more preferably 250 microns or less, most preferably 100 or 200 microns); rod shaped systems of 1-10 mm diameter, which generally are long and slender, may also be used.

The term therapeutic agent, as used herein, means any agent used to treat or prevent any disease or disorder of the body. Representative agents include hormones (and hormone fragments and analogues), e.g., testosterone, luteinizing hormone-releasing hormone (LHRH); diuretics, e.g., chlorothiazide; antiinflammatories; pain killers, e.g., morphine; antibiotics, e.g., tetracycline; antipsychotic drugs; anticancer drugs, e.g., methotrexate, actinomycin D, vinblastine, and cytosine arabinoside; vaccines; and antiarthritic drugs, e.g., ibuprofen and flurbiprofen.

Certain such agents, and others, may be administered to animals, such as animals grown for their milk, meat or skin products (e.g. wool or leather) other than for therapy or for diagnosing diseases in such animals.

One of the roles of the barrier substance is to limit the initial burst of agent from the delivery system. The barrier substance should not affect the long term, sustained release of the agent, and therefore should be a substance that dissolves or wears off the surface of the system within a short period of time after injection; preferably, the barrier substance should dissipate within 5 days after injection, more preferably within 2 days after injection. The substance should be non-toxic, non-irritating, non-sensitizing, and hydrophobic. Preferably the substance is on the GRAS list or is USP approved.

Examples of suitable barrier substances include paraffins, beeswax, and, preferably, silicone oils.

Silicone oils are organo-siloxane polymers based on a structure consisting of alterating silicon and oxygen atoms with organic groups (R) attached to the silicon atoms: 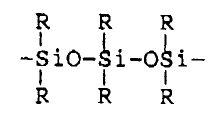
Typical silicone oils include those in which R is a lower alkyl group having six or less carbons, e.g., a methyl group. Silicone oils have a viscosity of between 1 cp and 10⁶cp. The more preferred silicone oils have a viscosity of between 10² cp and 10⁴ cp; most preferably the silicone oils have a viscosity of between 500 cp and 2000 cp. Representative examples of silicone oils include Union Carbide dimethylpolysilicone #L-45 (viscosity 1000 cp), Dow Corning medical grade silicone oil #360, Aldrich silicone oil (catalogue no. 17,563-3). and Aldrich silicone oil (catalogue no. 14,615-3).

The delivery system can comprise up to 70% agent by weight. If a system comprises greater than 70% agent by weight, the mechanical properties of the system may be adversely affected.

The uncoated delivery system can be prepared by standard solvent casting techniques. In general, the polymer is dissolved in an organic solvent and the therapeutic agent added. The solution or suspension is then poured into a suitable mould and the solvent evaporated to yield a polymer-agent combination. Alternatively, the agent can be dispersed with the polymer without solvent.

The delivery system can also be prepared by coacervation procedures, such as those described by Lapka et al., U.S. Pat. No. 4,622,244, the disclosure of which is hereby incorporated by reference.

The uncoated delivery system of choice (generally in the form of a powder) is placed in a flask and covered with a silicone oil such as Union Carbide dimethylpolysilicone #L-45. The thickness of the coating can be adjusted by rinsing with hexane or other organic solvent capable of dissolving silicone oil. The thicker the coating, the more limited the initial burst of release of agent from the system. The powder is dried under a stream of nitrogen gas, and then under vacuum for 24 hours.

Alternate methods of coating including spraying a thin coat of silicone oil onto the powder; and mixing the powder in a solvent in which the barrier substance has been dissolved and then evaporating off the solvent.

Where the delivery system is a powder made up of particles having an average size 100-200 microns, following coating each particle should be 0.05%-10% (more preferably 0.1%-2%) silicone oil by weight. The amount of silicone oil present can be calculated by first conducting an elemental analysis of the powder to determine the amount of silicon present; knowing the percentage of silicon in the oil, the amount of oil can then be determined.

The delivery systems containing the standard dosage of the selected therapeutic agent and coated with a barrier substance are injected, implanted, or otherwise inserted parenterally into a patient. The barrier substance limits the initial burst of the agent from the polymer; over a short period of time, the barrier substance wears off of the surface of the system, allowing the system to release the desired dosage over a sustained period of time.

A comparison of the initial quantity of agent released from the coated delivery system and an uncoated delivery system can be made by (1) injecting or implanting equal amounts (uncoated weight) of the systems into different animals; and (2) either determining the levels of agent in the animal's blood after 48 hours, or measuring the biological effect that the agent induces (e.g., for delivery systems that release LHRH, the level of testosterone in serum is measured, that level being directly correlated to the amount of LHRH released). It is understood that, although it is most preferred that the initial release be limited to the desired dosage of the agent, advantages, such as reducing side effects of large dosages of the agent, are achieved if the initial release is reduced by as little as ten percent (more preferably twenty percent).

## Claims (Claims for the following Contracting State(s): AT, BE, CH, DE, FR, GB, IT, LI, LU, NL, SE)

1. A delivery system adapted for delivery of an agent, such as a therapeutic agent, or other substance to a living person or animal by parenteral injection or implantation, comprising a biodegradable polymer and a therapeutic agent dispersed in said polymer, and being coated with a barrier substance that is effective, during a period of forty-eight hours immediately subsequent to parenteral injection or implantation of said system into a living person or animal, to decrease the quantity of said agent or substance released from said system, as compared with the quantity of said agent or substance released from a similar said system not so coated, during said period, said barrier substance being not susceptible to enzymatic attack and one which dissipates in said living person or animal substantially by wearing off from the surface of said delivery system.

2. A delivery system according to Claim 1, further characterised in that said barrier substance comprises a paraffin, beeswax or silicone oil.

3. A delivery system according to Claim 2, further characterised in that said barrier substance is a silicone oil.

4. A delivery system according to Claim 3, further characterised in that said silicone oil has a viscosity of between 10² centipoise and 10⁴ centipoise.

5. A delivery system according to Claim 4, further characterised in that said silicone oil has a viscosity of between 500 centipoise and 2000 centipoise.

6. A method of administering an agent or substance to a vertebrate other than during surgical treatment or in therapy or in a method of diagnosis of disease by the use of a delivery system comprising a biodegradable polymer and said agent or substance dispersed in said polymer, characterised in that it includes the steps of: coating said delivery system with a barrier substance; and injecting or implanting the coated delivery system into said vertebrate; said barrier substance being effective to limit the initial release of said agent or substance from said system in comparison with the initial release obtained from a similar said system not coated with said barrier substance, said barrier substance being not susceptible to enzymatic attack and one which dissipates in said living person or animal substantially by wearing off from the surface of said delivery system.

7. A method according to Claim 6, further characterized in that said barrier substance is a paraffin, beeswax or a silicone oil.

8. A method according to Claim 6, further characterised in that said barrier substance is a silicone oil.

9. A method according to Claim 8, further characterized in that said silicone oil has a viscosity of between 10² centipoise and 10⁴ centipoise.

10. A method according to Claim 9, further characterized in that said silicone oil has a viscosity of between 500 centipoise and 2000 centipoise.

11. A method of providing a delivery system adapted for delivery of an agent, such as a therapeutic agent, or other substance to a living person or animal by parenteral injection or implantation, characterized in comprising the steps of: coating a delivery system comprising a biodegradable polymer and said agent or substance dispersed within said polymer with a barrier substance effective, during a period of forty-eight hours immediately subsequent to the parenteral injection or implantation of said system into a living person or animal, to decrease the quantity of said agent or substance released from said system, as compared with the quantity of said agent or substance released from a similar said system not so coated during said period, said barrier substance being not susceptible to enzymatic attack and one which dissipates in said living person or animal substantially by wearing off from the surface of said delivery system; and treating said coated delivery system to remove some but not all of said barrier substance.

12. A method according to Claim 11, further characterized in that said barrier substance comprises a silicone oil, a paraffin, or beeswax.

13. A method according to Claims 11 or 12, further characterized in that said treating comprises washing said delivery system with a solvent.

## Claims (Claims for the following Contracting State(s): ES, GR)

1. A method of manufacturing a delivery system adapted for delivery of an agent, such as a therapeutic agent, or other substance to a living person or animal by parenteral injection or implantation, Comprising; providing a biodegradable polymer having a therapeutic agent dispersed therein, and coating said biodegradable polymer having said agent dispersed therein with a barrier substance that is effective, during a period of forty-eight hours immediately subsequent to parenteral injection or implantation of said system into a living person or animal, to decrease the quantity of said agent or substance released from said system, as compared with the quantity of said agent or substance released from a similar said system not so coated, during said period, said barrier substance being not susceptible to enzymatic attack and one which dissipates in said living person or animal substantially by wearing off from the surface of said delivery system.

2. A method according to Claim 1, further characterized in that said barrier substance comprises a paraffin, beeswax or silicone oil.

3. A method according to Claim 2, further characterized in that said barrier substance is a silicone oil.

4. A method according to Claim 3, further characterized in that said Silicone oil has a viscosity of between 10² centipoise and 10⁴ centipoise.

5. A method according to Claim 4, further characterized in that said silicone oil has a viscosity of between 500 centipoise and 2000 centipoise.

6. A method of administering an agent or substance to a vertebrate other than during surgical treatment or in therapy or in a method of diagnosis of disease by the use of a delivery system comprising a biodegradable polymer and said agent or substance dispersed in said polymer, characterised in that it includes the steps of: coating said delivery system with a barrier substance; and injecting or implanting the coated delivery system into said vertebrate; said barrier substance being effective to limit the initial release of said agent or substance from said system in comparison with the initial release obtained from a similar said system not coated with said barrier substance, said barrier substance being not susceptible to enzymatic attack and one which dissipates in said living person or animal substantially by wearing off from the surface of said delivery system.

7. A method according to Claim 6, further characterised in that said barrier substance is a paraffin, beeswax or a silicone oil.

8. A method according to Claim 6, further characterised in that said barrier substance is a silicone oil.

9. A method according to Claim 8, further characterized in that said silicone oil has a viscosity of between 10² centipoise and 10⁴ centipoise.

10. A method according to Claim 9, further characterized in that said silicone oil has a viscosity of between 500 centipoise and 2000 centipoise.

11. A method of providing a delivery system adapted for delivery of an agent, such as a therapeutic agent, or other substance to a living person or animal by parenteral injection or implantation, characterized in comprising the steps of: coating a delivery system comprising a biodegradable polymer and said agent or substance dispersed within said polymer with a barrier substance effective, during a period of forty-eight hours immediately subsequent to the parenteral injection or implantation of said system into a living person or animal, to decrease the quantity of said agent or substance released from said system, as compared with the quantity of said agent or substance released from a similar said system not so coated during said period, said barrier substance being not susceptible to enzymatic attack and one which dissipates in said living person or animal substantially by wearing off from the surface of said delivery system; and treating said coated delivery system to remove some but not all of said barrier substance.

12. A method according to Claim 11, further characterised in that said barrier substance comprises a silicone oil, a paraffin or a beeswax.

13. A method according to Claim 11 or 12, further characterised in that said treating step comprises washing said delivery system with a solvent.

## Patentansprüche (Patentansprüche für folgende(n) Vertragsstaat(en): AT, BE, CH, DE, FR, GB, IT, LI, LU, NL, SE)

1. Abgabesystem, das zur Abgabe eines Mittels, wie eines therapeutischen Mittels, oder einer anderen Substanz an eine lebende Person oder an ein Tier durch parenterale Injektion oder Implantation geeignet ist, umfassend ein biologisch abbaubares Polymer und ein in dem genannten Polymeren dispergiertes therapeutisches Mittel, und das mit einer Barriere-substanz überzogen ist, die wahrend eines Zeitraums von 48 Stunden unmittelbar im Anschluß an die parenterale Injektion oder Implantation des genannten Systems in eine lebende Person oder in ein Tier wirksam ist, um die Menge des genannten Mittels oder der Substanz, die von dem genannten System freigesetzt wird, im Vergleich zu der Menge des genannten Mittels oder der Substanz, die aus einem ähnlichen, nicht so beschichteten System freigesetzt wird, während des genannten Zeitraumes zu verringern, wobei bei die genannten Barrieresubstanz gegenüber einem enzymatischen Angriff nicht empfindlich ist, und eine ist, die sich in der genannten lebenden Person oder dem Tier im wesentlichen durch Abnutzung von der Oberfläche des genannten Abgabesystems verteilt.

2. Abgabesystem nach Anspruch 1, das außerdem dadurch gekennzeichnet ist, daß die genannte Barrieresubstanz ein Paraffin, Bienenwachs oder Siliconöl umfaßt.

3. Abgabesystem nach Anspruch 1, das außerdem dadurch gekennzeichnet ist, daß die genannte Barrieresubstanz ein Siliconöl ist.

4. Abgabesystem nach Anspruch 3, das außerdem dadurch gekennzeichnet ist, daß das genannte Siliconöl eine Viskosität zwischen 10² Centipoise und 10⁴ Centipoise aufweist.

5. Abgabesystem nach Anspruch 4, das außerdem dadurch gekennzeichnet ist, daß das genannte Siliconöl eine Viskosität zwischen 500 Centipoise und 2000 Centipoise aufweist.

6. Verfahren zur Verabreichung eines Mittels oder einer Substanz an einen Vertebraten, außer bei einer operativen Behandlung oder einer Therapie oder bei einem Verfahren zur Krankeitsdiagnose, unter Verwendung eines Abgabesystems, das ein biologisch abbaubares Polymer und das genannte Mittel oder die Substanz dispergiert in dem genannten Polymeren umfaßt, dadurch gekennzeichnet, daß es die folgenden Stufen einschließt: Überziehen des genannten Polymeren umfaßt, dadurch gekennzeichnet, daß es die folgenden Stufen einschließt: Überziehen des genannten Abgabesystems mit einer Barrieresubstanz, Injektion oder Implantation des überzogenen Abgabesystems in den genannten Vertebraten, wobei die genannte Barrieresubstanz wirksam ist, um die anfängliche Freitsetzung des genannten Mittels oder der genannten Substanz aus dem genannten System im Vergleich mit der anfänglichen Freitsetzung, die aus einem ähnlichen System, das nicht mit der genannten Barrieresubstanz überzogen ist, erhalten wird, zu verringern, wobei die genannte Barrieresubstanz gegenüber einem enzymatischen Angriff nicht empfindlich ist und eine ist, die sich in der genannten lebenden Person oder dem Tier im wesentlichen durch Abnutzung von der Oberfläche des genannten Abgabesystems verteilt.

7. Verfahren nach Anspruch 6, das außerdem dadurch gekennzeichnet ist, daß die genannte Barrieresubstanz ein Paraffin, Bienenwachs oder ein Siliconöl ist.

8. Verfahren nach Anspruch 6, das außerdem dadurch gekennzeichnet ist, daß die genannte Barrieresubstanz ein Siliconöl ist.

9. Verfahren nach Anspruch 8, das außerdem dadurch gekennzeichnet ist, daß das genannte Siliconöl eine Viskosität zwischen 10² Centipoise und 10⁴ Centipoise aufweist.

10. Verfahren nach Anspruch 9, das außerdem dadurch gekennzeichnet ist, daß das genannte Siliconöl eine Viskosität zwischen 500 Centipoise und 2000 Centipoise aufweist.

11. Verfahren zur Bereitstellung eines Abgabesystems, das zur Freitsetzung eines Mittels, wie eines therapeutischen Mittels oder einer weiteren Substanz, an eine lebende Person oder an ein Tier durch parenterale Injektion oder Implantation geeignet ist, dadurch gekennzeichnet, daß es die folgenden Stufen umfaßt: Überziehen eines Abgabesystems, das ein biologisch abbaubares Polymeres und das genannte Mittel oder die genannte Substanz, dispergiert im dem genannten Polymeren, mit einer Barrieresubstanz, die während eines Zeitraumes von 48 Stunden unmittelbar im Anschluß an die parenterale Injektion oder Implantation des genannten Systems an eine lebende Person oder an ein Tier, einschließt, um die Menge des genannten Mittels oder der Substanz, die aus dem genannten System freigesetzt wird im Vergleich zu der Menge des genannten Mittels oder der genannten Substanz, die aus einem ähnlichen nicht so beschichteten genannten System während des genannten Zeitraums freigesetzt werden, zu verringern, wobei die genannte Barrieresubstanz gegenüber einem enzymatischen Angriff nicht empfindlich ist, und eine ist, die sich in der genannten lebenden Person oder dem Tier im wesentlichen durch Abnutzung von der Oberfläche des genannten Abgabesystems verteilt, und Behandeln des genannten überzogenen Abgabesystems, um einen Teil, jedoch nicht alles, der genannten Barrieresubstanz zu entfernen.

12. Verfahren nach Anspruch 11, das außerdem dadurch gekennzeichnet ist, daß die genannte Barrieresubstanz ein Siliconöl, ein Paraffin oder Bienenwachs umfaßt.

13. Verfahren nach Anspruch 11 oder 12, das außerdem dadurch gekennzeichnet ist, daß die genannte Behandlung das Waschen des genannten Abgabesystems mit einem Lösungsmittel umfaßt.

## Patentansprüche (Patentansprüche für folgende(n) Vertragsstaat(en): ES, GR)

1. Verfahren zur Herstellung eines Abgabesystems, das zur Abgabe eines Mittels, wie eines therapeutischen Mittels, oder einer anderen Substanz an eine lebende Person oder an ein Tier durch parenterale Injektion oder Implantation geeignet ist, umfassend ein biologisch abbaubares Polymer und ein in dem genannten Polymeren dispergiertes therapeutisches Mittel, und das mit einer Barriere-substanz überzogen ist, die während eines Zeitraums von 48 Stunden unmittelbar im Anschluß an die parenterale Injektion oder Implantation des genannten Systems in eine lebende Person oder in ein Tier wirksam ist, um die Menge des genannten Mittels oder der Substanz, die von dem genannten System freigesetzt wird, im Vergleich zu der Menge des genannten Mittels oder der Substanz, die aus einem ähnlichen, nicht so beschichteten System freigesetzt wird, während des genannten Zeitraumes zu verringern, wobei bei die genannten Barrieresubstanz gegenüber einem enzymatischen Angriff nicht empfindlich ist, und eine ist, die sich in der genannten lebenden Person oder dem Tier im wesentlichen durch Abnutzung von der Oberfläche des genannten Abgabesystems verteilt.

2. Verfahren nach Anspruch 1, das außerdem dadurch gekennzeichnet ist, daß die genannte Barrieresubstanz ein Paraffin, Bienenwachs oder Siliconöl umfaßt.

3. Verfahren nach Anspruch 1, das außerdem dadurch gekennzeichnet ist, daß die genannte Barrieresubstanz ein Siliconöl ist.

4. Verfahren nach Anspruch 3, das außerdem dadurch gekennzeichnet ist, daß das genannte Siliconöl eine Viskosität zwischen 10² Centipoise und 10⁴ Centipoise aufweist.

5. Verfahren nach Anspruch 4, das außerdem dadurch gekennzeichnet ist, daß das genannte Siliconöl eine Viskosität zwischen 500 Centipoise und 2000 Centipoise aufweist.

6. Verfahren zur Verabreichung eines Mittels oder einer Substanz an einen Vertebraten, außer bei einer operativen Behandlung oder einer Therapie oder bei einem Verfahren zur Krankeitsdiagnose, unter Verwendung eines Abgabesystems, das ein biologisch abbaubares Polymer und das genannte Mittel oder die Substanz dispergiert in dem genannten Polymeren umfaßt, dadurch gekennzeichnet, daß es die folgenden Stufen einschließt: Überziehen des genannten Polymeren umfaßt, dadurch gekennzeichnet, daß es die folgenden Stufen einschließt: Überziehen des genannten Abgabesystems mit einer Barrieresubstanz, Injektion oder Implantation des überzogenen Abgabesystems in den genannten Vertebraten, wobei die genannte Barrieresubstanz wirksam ist, um die anfängliche Freitsetzung des genannten Mittels oder der genannten Substanz aus dem genannten System im Vergleich mit der anfänglichen Freitsetzung, die aus einem ähnlichen System, das nicht mit der genannten Barrieresubstanz überzogen ist, erhalten wird, zu verringern, wobei die genannte Barrieresubstanz gegenüber einem enzymatischen Angriff nicht empfindlich ist und eine ist, die sich in der genannten lebenden Person oder dem Tier im wesentlichen durch Abnutzung von der Oberfläche des genannten Abgabesystems verteilt.

7. Verfahren nach Anspruch 6, das außerdem dadurch gekennzeichnet ist, daß die genannte Barrieresubstanz ein Paraffin, Bienenwachs oder ein Siliconöl ist.

8. Verfahren nach Anspruch 6, das außerdem dadurch gekennzeichnet ist, daß die genannte Barrieresubstanz ein Siliconöl ist.

9. Verfahren nach Anspruch 8, das außerdem dadurch gekennzeichnet ist, daß das genannte Siliconöl eine Viskosität zwischen 10² Centipoise und 10⁴ Centipoise aufweist.

10. Verfahren nach Anspruch 9, das außerdem dadurch gekennzeichnet ist, daß das genannte Siliconöl eine Viskosität zwischen 500 Centipoise und 2000 Centipoise aufweist.

11. Verfahren zur Bereitstellung eines Abgabesystems, das zur Freitsetzung eines Mittels, wie eines therapeutischen Mittels oder einer weiteren Substanz, an eine lebende Person oder an ein Tier durch parenterale Injektion oder Implantation geeignet ist, dadurch gekennzeichnet, daß es die folgenden Stufen umfaßt: Überziehen eines Abgabesystems, das ein biologisch abbaubares Polymeres und das genannte Mittel oder die genannte Substanz, dispergiert im dem genannten Polymeren, mit einer Barrieresubstanz, die während eines Zeitraumes von 48 Stunden unmittelbar im Anschluß an die parenterale Injektion oder Implantation des genannten Systems an eine lebende Person oder an ein Tier, einschließt, um die Menge des genannten Mittels oder der Substanz, die aus dem genannten System freigesetzt wird im Vergleich zu der Menge des genannten Mittels oder der genannten Substanz, die aus einem ähnlichen nicht so beschichteten genannten System während des genannten Zeitraums freigesetzt werden, zu verringern, wobei die genannte Barrieresubstanz gegenüber einem enzymatischen Angriff nicht empfindlich ist, und eine ist, die sich in der genannten lebenden Person oder dem Tier im wesentlichen durch Abnutzung von der Oberfläche des genannten Abgabesystems verteilt, und Behandeln des genannten überzogenen Abgabesystems, um einen Teil, jedoch nicht alles, der genannten Barrieresubstanz zu entfernen.

12. Verfahren nach Anspruch 11, das außerdem dadurch gekennzeichnet ist, daß die genannte Barrieresubstanz ein Siliconöl, ein Paraffin oder Bienenwachs umfaßt.

13. Verfahren nach Anspruch 11 oder 12, das außerdem dadurch gekennzeichnet ist, daß die genannte Behandlung das Waschen des genannten Abgabesystems mit einem Lösungsmittel umfaßt.

## Revendications (Revendications pour l'(les) Etat(s) contractant(s) suivant(s): AT, BE, CH, DE, FR, GB, IT, LI, LU, NL, SE)

1. Un système d'administration adapté pour l'administration d'un agent tel qu'un agent thérapeutique ou une autre substance à une personne ou un animal vivant(e), par injection ou implantation parentérale, comprenant un polymère biodégradable et un agent thérapeutique dispersé dans ledit polymère, et revêtu d'une substance-barrière qui est efficace pendant une période de quarante-huit heures immédiatement après l'injection ou l'implantation parentérale dudit système chez une personne ou un animal vivant(e), afin de diminuer la quantité dudit agent ou de la dite substance libéré(e) dudit système, en comparaison de la quantité dudit agent ou de ladite substance libéré(e) d'un dit système semblable non revêtu de la sorte, pendant ladite période, ladite substance-barrière n'étant pas sensible à une attaque enzymatique et étant une substance qui se dissipe dans ladite personne ou ledit animal vivant(e) substantiellement par érosion progressive de la surface dudit système d'administration.

2. Un système d'administration selon la revendication 1, caractérisé en outre par le fait que ladite substance-barrière comprend une paraffine, de la cire d'abeilles ou une huile de silicone.

3. Un système d'administration selon la revendication 2, caractérisé en outre par le fait que ladite substance-barrière est une huile de silicone.

4. Un système d'administration selon la revendication 3, caractérisé en outre par le fait que ladite huile de silicone a une viscosité entre 10² centipoises et 10⁴ centipoises.

5. Un système d'administration selon la revendication 4, caractérisé en outre par le fait que ladite huile de silicone a une viscosité entre 500 centipoises et 2000 centipoises.

6. Une méthode d'administration d'un agent ou d'une substance à un vertébré, autrement que pendant un traitement chirurgical ou dans le cadre d'une thérapie ou dans le cadre d'une méthode de diagnostic de maladie, par l'utilisation d'un système d'administration comprenant un polymère biodégradable et ledit agent ou ladite substance dispersé(e) dans ledit polymère, caractérisée par le fait qu'elle comprend les phases suivantes : revêtement dudit système d'administration avec une substance-barrière; et injection ou implantation du système d'administration revêtu chez ledit vertébré; ladite substance-barrière étant efficace pour limiter la libération initiale dudit agent ou de ladite substance, dudit système, en comparaison de la libération initiale obtenue d'un dit système semblable non revêtu avec ladite substance-barrière, ladite substance-barrière n'étant pas sensible à une attaque enzymatique et étant une substance qui se dissipe dans ladite personne ou ledit animal vivant(e) substantiellement par érosion progressive, de la surface dudit système d'administration.

7. Une méthode selon la revendication 6, caractérisée en outre par le fait que ladite substance-barrière est une paraffine, de la cire d'abeille ou une huile de silicone.

8. Une méthode selon la revendication 6, caractérisée en outre par le fait que ladite substance-barrière est une huile de silicone.

9. Une méthode selon la revendication 8, caractérisée en outre par le fait que ladite huile de silicone a une viscosité entre 10² centipoise et 10⁴ centipoises.

10. Une méthode selon la revendication 9, caractérisée en outre par le fait que ladite huile de silicone a une viscosité entre 500 centipoises et 2000 centipoises.

11. Une méthode d'obtention d'un système d'administration adapté pour l'administration d'un agent tel qu'un agent thérapeutique ou une autre substance à une personne ou un animal vivant(e), par injection ou implantation parentérale, caractérisée par le fait qu'elle comprend les phases suivantes : revêtement d'un système d'administration comprenant un polymère biodégradable et ledit agent ou ladite substance dispersé(e) à l'intérieur dudit polymère avec une substance-barrière efficace pendant une période de quarante-huit heures immédiatement après l'injection ou l'implantation parentérale dudit système chez une personne ou un animal vivant(e) afin de diminuer la quantité dudit agent ou de ladite substance libéré(e) dudit système, en comparaison de la quantité dudit agent ou de ladite substance libéré(e) d'un dit système semblable non revêtu de la sorte pendant ladite période, ladite substance-barrière n'étant pas sensible à une attaque enzymatique et étant une substance qui se dissipe dans ladite personne ou ledit animal vivant(e) substantiellement par érosion progressive, de la surface dudit système d'administration ; et traitement dudit système d'administration revêtu pour éliminer seulement en partie ladite substance-barrière.

12. Une méthode selon la revendication 11, caractérisée en outre par le fait que ladite substance-barrière comprend une huile de silicone, une paraffine ou de la cire d'abeilles.

13. Une méthode selon la revendication 11 ou 12, caractérisée en outre par le fait que ledit traitement comprend le lavage dudit système d'administration avec un solvant.

## Revendications (Revendications pour l'(les) Etat(s) contractant(s) suivant(s): ES, GR)

1. Une méthode d'obtention d'un système d'administration adapté pour l'administration d'un agent, tel qu'un agent thérapeutique ou une autre substance à une personne ou un animal vivant(e), par injection ou implantation parentérale, comprenant ; l'emploi d'un polymère-biodégradable dans lequel un agent thérapeutique a été dispersé, et le revêtement dudit polymère biodégradable contenant l'agent ainsi dispersé avec une substance-barrière qui est efficace, pendant une période de quarantehuit heures immédiatement après l'injection ou l'implantation parentérale dudit système chez une personne ou un animal vivant(e), afin de diminuer la quantité dudit agent ou de la dite substance libéré(e) dudit système, en comparaison de la quantité dudit agent ou de ladite substance libéré(e) d'un dit système semblable non revêtu de la sorte, pendant ladite période, ladite substance-barrière n'étant pas sensible à une attaque enzymatique et étant une substance qui se dissipe dans ladite personne ou ledit animal vivant(e) substantiellement par érosion progressive de la surface dudit système d'administration.

2. Une méthode selon la revendication 1, caractérisé en outre par le fait que ladite substance-barrière comprend une paraffine, de la cire d'abeilles ou une huile de silicone.

3. Une méthode selon la revendication 2, caractérisé en outre par le fait que ladite substance-barrière est une huile de silicone.

4. Une méthode selon la revendication 3, caractérisé en outre par le fait que ladite huile de silicone a une viscosité entre 10² centipoises et 10⁴ centipoises.

5. Une méthode selon la revendication 4, caractérisé en outre parle fait que ladite huile de silicone a une viscosité entre 500 centipoises et 2000 centipoises.

6. Une méthode d'administration d'un agent ou d'une substance à un vertébré, autrement que pendant un traitement chirurgical ou dans le cadre d'une thérapie ou dans le cadre d'une méthode de diagnostic de maladie, par l'utilisation d'un système d'administration comprenant un polymère biodégradable et ledit agent ou ladite substance dispersé(e) dans ledit polymère, caractérisée par le fait qu'elle comprend les phases suivantes : revêtement dudit système d'administration avec une substance-barrière; et injection ou implantation du système d'administration revêtu chez ledit vertébré; ladite substance-barrière étant efficace pour limiter la libération initiale dudit agent ou de ladite substance, dudit système, en comparaison de la libération initiale obtenue d'un dit système semblable non revêtu avec ladite substance-barrière, ladite substance-barrière n'étant pas sensible à une attaque enzymatique et étant une substance qui se dissipe dans ladite personne ou ledit animal vivant(e) substantiellement par érosion progressive, de la surface dudit système d'administration.

7. Une méthode selon la revendication 6, caractérisée en outre par le fait que ladite substance-barrière est une paraffine, de la cire d'abeille ou une huile de silicone.

8. Une méthode selon la revendication 6, caractérisée en outre par le fait que ladite substance-barrière est une huile de silicone.

9. Une méthode selon la revendication 8, caractérisée en outre par le fait que ladite huile de silicone a une viscosité entre 10² centipoise et 10⁴ centipoises.

10. Une méthode selon la revendication 9, caractérisée en outre par le fait que ladite huile de silicone a une viscosité entre 500 centipoises et 2000 centipoises.

11. Une méthode d'obtention d'un système d'administration adapté pour l'administration d'un agent tel qu'un agent thérapeutique ou une autre substance à une personne ou un animal vivant(e), par injection ou implantation parentérale, caractérisée par le fait qu'elle comprend les phases suivantes : revêtement d'un système d'administration comprenant un polymère biodégradable et ledit agent ou ladite substance dispersé(e) à l'intérieur dudit polymère avec une substance-barrière efficace pendant une période de quarante-huit heures immédiatement après l'injection ou l'implantation parentérale dudit système chez une personne ou un animal vivant(e) afin de diminuer la quantité dudit agent ou de ladite substance libéré(e) dudit système, en comparaison de la quantité dudit agent ou de ladite substance libéré(e) d'un dit système semblable non revêtu de la sorte pendant ladite période, ladite substance-barrière n'étant pas sensible à une attaque enzymatique et étant une substance qui se dissipe dans ladite personne ou ledit animal vivant(e) substantiellement par érosion progressive, de la surface dudit système d'administration ; et traitement dudit système d'administration revêtu pour éliminer seulement en partie ladite substance-barrière.

12. Une méthode selon la revendication 11, caractérisée en outre par le fait que ladite substance-barrière comprend une huile de silicone, une paraffine ou de la cire d'abeilles.

13. Une méthode selon la revendication 11 ou 12, caractérisée en outre par le fait que ledit traitement comprend le lavage dudit système d'administration avec un solvant.
